# EUROPEAN PATENT APPLICATION

(11) **EP 4 725 507 A1**
(43) Date of publication of application: **15.04.2026**
(21) Application number: 24830498.2
(22) Date of filing: 12.06.2024
(51) Int. Cl.: A61K 47/64, A61K 31/337, A61P 35/00, C07K 7/06

(54) **POLYPEPTIDE CONJUGATE, PHARMACEUTICAL COMPOSITION CONTAINING POLYPEPTIDE CONJUGATE AND USE THEREOF**

(30) Priority: 25.06.2023 WO PCT/CN2023/102220
(71) Applicant: Nanjing Anji Biotechnology Co., Ltd., Nanjing, Jiangsu 210000 (CN)
(72) Inventor: XU, Hanmei, Nanjing, Jiangsu 210000 (CN); ZHANG, Hui, Nanjing, Jiangsu 210000 (CN); LIU, Chen, Nanjing, Jiangsu 210000 (CN)
(74) Representative: Prüfer & Partner mbB Patentanwälte · Rechtsanwälte
(86) International application number: PCT/CN2024/098760
(87) International publication number: WO 2025/001852

(57) **Abstract**

A polypeptide conjugate having the structure of formula (I): polypeptide-linker-compound, Formula (I), where the polypeptide is a peptide targeting a mesenchymal-epithelial transition factor, and the compound is a taxane compound or an enantiomer, a diastereoisomer, a solvate or a pharmaceutically acceptable salt thereof. A method for preparing the polypeptide conjugate; a pharmaceutical composition containing the polypeptide conjugate; a use of the polypeptide conjugate in the preparation of a drug for preventing or treating diseases; and a method for preventing or treating diseases using the polypeptide conjugate.

## Description

### CROSS-REFERENCE TO RELATED APPLICATIONS

The present application claims priority to the application PCT/CN2023/102220 filed on June 25, 2023. The prior application is deemed as a part of the disclosure of the present application and is incorporated herein in its entirety.

### TECHNICAL FIELD

The present application relates to a polypeptide conjugate, a pharmaceutical composition comprising the polypeptide conjugate, and a use thereof.

### BACKGROUND

In early 2021, the International Agency for Research on Cancer of the World Health Organization released the latest global cancer data for 2020, which showed that China ranked first in the world in both the number of new cancer cases and cancer deaths.

Currently, the main methods for treating cancer include surgical treatment, chemotherapy, and radiotherapy. In chemotherapy, taxane drugs such as docetaxel and paclitaxel are classic microtubule protein inhibitors, which can inhibit tumor cell mitosis and trigger apoptosis by inhibiting microtubule depolymerization. Paclitaxel is regarded as one of the most widely used natural anticancer drugs and has been widely applied in the treatment of breast cancer, colorectal cancer, bladder squamous cell carcinoma, head and neck cancer, small-cell lung cancer, non-small-cell lung cancer, and other diseases. Docetaxel has a broad antitumor spectrum and can be used in the treatment of advanced breast cancer, ovarian cancer, non-small-cell lung cancer, head and neck cancer, pancreatic cancer, small-cell lung cancer, gastric cancer, melanoma, soft tissue sarcoma, and other diseases.

However, taxane drugs such as paclitaxel and docetaxel cause significant damage to normal cells. With their extensive clinical applications, the incidence of adverse reactions has increased markedly, their dosage has been greatly limited, and the therapeutic effect has also been unsatisfactory. Meanwhile, taxane drugs such as paclitaxel and docetaxel are almost insoluble in water, which limits their clinical application.

Therefore, it is of great significance to improve the efficacy of a taxane anti-tumor drug and reduce its toxic and side effects.

### SUMMARY

The present application provides a polypeptide conjugate that covalently links a specific peptide sequence to a compound via a linker, selectively delivering the compound to target cells, thereby enhancing the efficacy of the cytotoxic compound and reducing systemic toxicity. Further, through conjugation with the polypeptide of the present application, the polypeptide conjugate and the pharmaceutical composition of the present application exhibit excellent water solubility, which enhances the water solubility of taxane drugs such as paclitaxel and docetaxel.

In a first aspect, the present application provides a polypeptide conjugate having the structure of Formula (I):

polypeptide-linker-compound Formula (I)

where,
the polypeptide is a mesenchymal-epithelial transition factor targeting peptide, and
the compound is a taxane compound or an enantiomer, a diastereoisomer, a solvate or a pharmaceutically acceptable salt thereof.

Mesenchymal-epithelial transition factor (c-Met) is a tyrosine kinase receptor activated by hepatocyte growth factor, which regulates multiple biological processes. The c-Met targeting peptide can specifically bind to various c-Met-positive tumor cells such as those of gastric cancer, liver cancer, lung cancer, and colorectal cancer. Therefore, the taxane compound coupled with the c-Met targeting polypeptide has favorable targeting properties, which can enhance the efficacy of the taxane compound, reduce its dosage, and mitigate its toxic and side effects, thereby enabling more effective tumor treatment.

In a second aspect, the present application provides a pharmaceutical composition comprising the polypeptide conjugate as described in the present application, and a pharmaceutically acceptable carrier.

In a third aspect, the present application provides a use of the polypeptide conjugate as described in the present application or the pharmaceutical composition as described in the present application in the preparation of a drug for preventing or treating diseases, optionally, the diseases comprise tumors, more optionally, the tumors comprise one or more of the group consisting of thyroid cancer, esophageal cancer, lung cancer, non-small cell lung cancer, gastric cancer, liver cancer, cholangiocarcinoma, renal cancer, pancreatic cancer, intestinal cancer, colorectal cancer, bladder cancer, prostate cancer, breast cancer, uterine cancer, endometrial cancer, ovarian cancer, cervical cancer, fallopian tube cancer, skin cancer, brain tumor, glioma, glioblastoma, melanoma, osteosarcoma, sarcoma, lymphoma, Hodgkin's lymphoma, non-Hodgkin's lymphoma, multiple myeloma, leukemia, acute lymphoblastic leukemia, chronic lymphocytic leukemia, acute myeloid leukemia, or chronic myeloid leukemia.

### BRIEF DESCRIPTION OF THE DRAWINGS

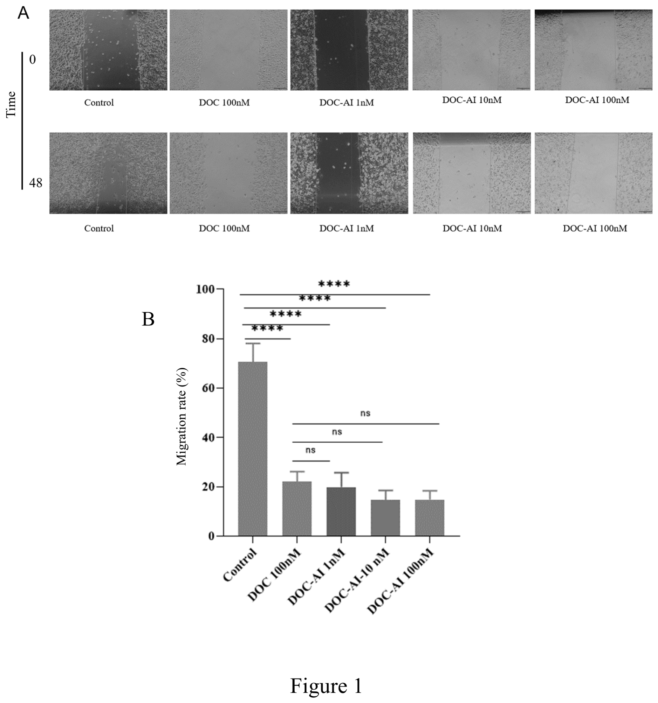

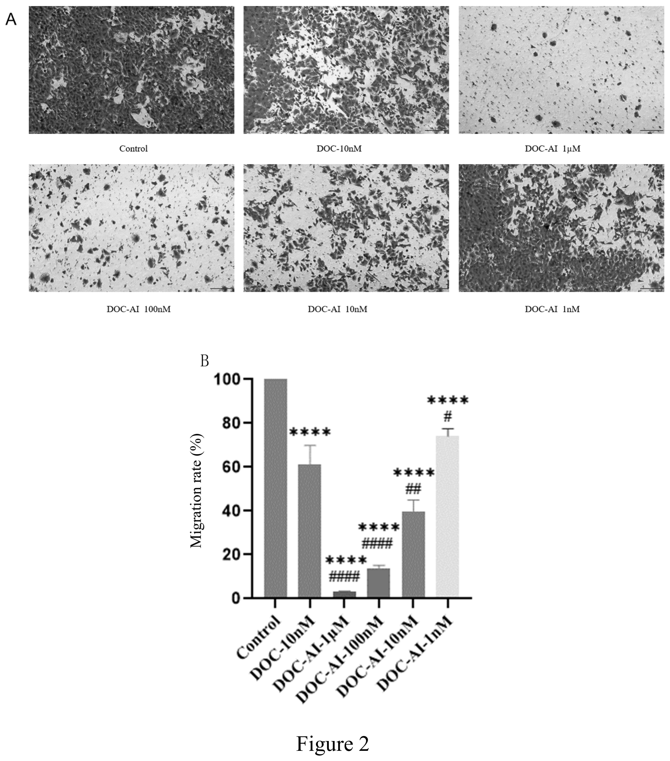

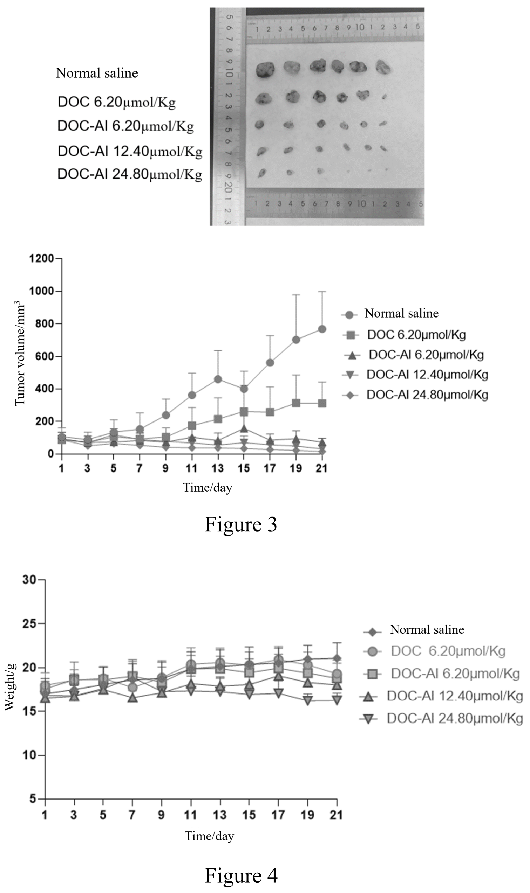

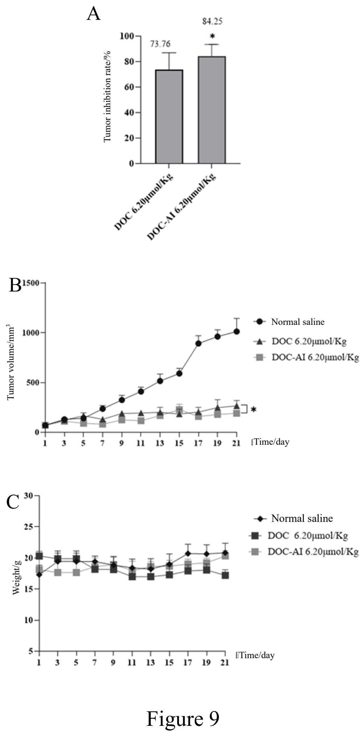

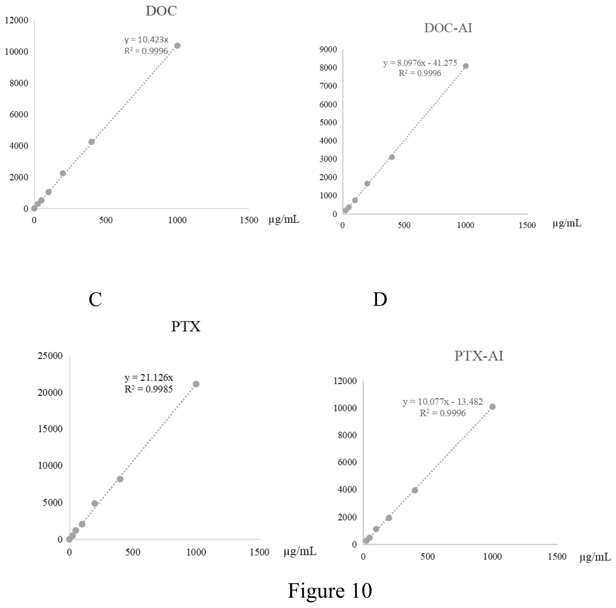

The following provides a brief description of the drawings, which are intended to illustrate the exemplary embodiments disclosed herein and not to limit these embodiments.
A of Figure. 1 shows the horizontal migration images of tumor cells in Example 3, specifically illustrating the horizontal migration images of tumor cells in the blank group, the positive control group and the administration group at 0 h and 48 h; B of Figure. 1 shows the statistical graph of horizontal migration rate of tumor cells in Example 3, specifically illustrating the statistical graph of horizontal migration rate of tumor cells in the blank group, the positive control group and the administration group.
A of Figure. 2 shows the vertical migration images of tumor cells in Example 4, specifically illustrating the vertical migration images of tumor cells in the blank group, the positive control group and the administration group at 48 h; B of Figure. 2 shows the statistical graph of vertical migration rate of tumor cells in Example 4, specifically illustrating the statistical graph of vertical migration rate of tumor cells in the blank group, the positive control group and the administration group.
A of Figure. 3 shows the visual diagram of tumor volume changes in nude mice inoculated with non-small cell lung cancer A549 cells after treatment with normal saline, DOC, and DOC-AI at different concentrations, respectively in Example 5; B of Figure. 3 shows the curve diagram of tumor volume changes in nude mice inoculated with non-small cell lung cancer A549 cells after treatment with normal saline, DOC, and DOC-AI at different concentrations, respectively in Example 5.
Figure. 4 shows the curve diagram of body weight changes of nude mice inoculated with non-small cell lung cancer A549 cells after treatment with normal saline, DOC, and DOC-AI at different concentrations, respectively in Example 5.
Figure. 5 shows the statistical graph of tumor weights in nude mice inoculated with non-small cell lung cancer A549 cells after treatment with normal saline, DOC, and DOC-AI at different concentrations, respectively in Example 5.
Figure. 6 shows the graph of tumor volume changes in nude mice inoculated with gastric cancer cells MKN-45 after treatment with normal saline, PTX, and PTX-AI at different concentrations, respectively. in Example 5
Figure. 7 shows the curve diagram of hepatic microsomal metabolism of DOC-AI in Example 6.
Figure. 8 shows the curve diagram of the hepatic microsomal metabolism of PTX-AI in Example 6.
A of Figure. 9 shows the statistical graph of tumor inhibition rate in nude mice inoculated with gastric cancer cells MKN-45 after treatment with DOC and DOC-AI in Example 5; B of Figure. 9 shows the curve diagram of tumor volume changes in nude mice inoculated with gastric cancer cells MKN-45 after treatment with normal saline, DOC, and DOC-AI in Example 5; C of Figure. 9 shows the curve diagram of body weight changes in nude mice inoculated with gastric cancer cells MKN-45 after treatment with normal saline, DOC, and DOC-AI in Example 5,.
A of Figure. 10 shows the concentration-peak area standard curve of the DOC standard solution in Example 7; B of Figure. 10 shows the concentration-peak area standard curve of the DOC-AI standard solution in Example 7; C of Figure. 10 shows the concentration-peak area standard curve of the PTX standard solution in Example 7; D of Figure. 10 shows the concentration-peak area standard curve of the PTX-AI standard solution in Example 7.

### DETAILED DESCRIPTION OF THE EMBODIMENTS

Unless otherwise indicated, all numbers representing content, concentration, ratio, weight, particle diameter, percentage, technical effect, and so forth as used in the specification and claims are to be understood as being modified in any case by the term "about" or "approximately". Accordingly, unless indicated to the contrary, numerical parameters set forth in the following specification and attached claims are approximations.

Unless otherwise specified, the terms used herein have the meanings commonly understood by those skilled in the pertinent technical field. For those skilled in the art, each numerical parameter may vary depending upon the desired properties and effects sought to be obtained by the present disclosure and should be construed in light of the significant figures of digits and ordinary rounding techniques or in a manner understood by those skilled in the art. Generally speaking, the naming used herein and the experimental operations for organic chemistry, pharmaceutical chemistry, and biology described herein are well-known in the art and widely adopted in the art. Unless otherwise defined, all technical and scientific terms used herein generally have the same meanings as those commonly understood by those of ordinary skill in the art to which the present disclosure belongs. In the case of multiple definitions of the terms used herein, unless otherwise specified, the definitions in this section shall prevail.

When used herein, the expression "A and/or B" includes three cases: (1) A; (2) B; and (3) A and B. The expression "A, B and/or C" includes seven cases: (1) A; (2) B; (3) C; (4) A and B; (5) A and C; (6) B and C; and (7) A, B and C. The meaning of similar expressions can be deduced in the same way.

### Polypeptide Conjugate

In a first aspect, the present application provides a polypeptide conjugate having the structure of Formula (I):

polypeptide-linker-compound Formula (I)

where,
the polypeptide is a mesenchymal-epithelial transition factor targeting peptide or a derivative thereof,
the compound is a taxane compound or an enantiomer, a diastereoisomer, a solvate or a pharmaceutically acceptable salt thereof.

As used herein, the term "mesenchymal-epithelial transition factor" is a tyrosine kinase receptor activated by hepatocyte growth factor, which regulates various biological processes and can be denoted as c-Met.

As used herein, unless otherwise specified or inconsistent with the context, the terms "polypeptide", "peptide" or "targeting peptide" encompass the referenced peptide itself, as well as its pharmaceutically acceptable salts, prodrugs and metabolites.

As used herein, the term "pharmaceutically acceptable salts" includes acid addition salts and base addition salts. Suitable acid addition salts are formed from acids that form non-toxic salts. Examples thereof include but are not limited to: acetate, adipate, aspartate, benzoate, benzenesulfonate, bicarbonate/carbonate, bisulfate/sulfate, borate, camphor sulfonate, citrate, cyclohexylamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002).

In some embodiments, the term "prodrug" refers to any molecule that is converted into the peptide of Formula I or a derivative thereof under physiological conditions in vivo, for example, through reactions such as oxidation, reduction, or hydrolysis under the action of enzymes, gastric acid, etc.

As used herein, the term "metabolite" refers to any molecule derived from the peptide of Formula I or a derivative thereof in a cell or organism (preferably a human).

As used herein, the term "enantiomer" refers to stereoisomers of two compounds that have the same planar structure and are non-superimposable mirror images of each other in terms of stereostructure.

As used herein, the term "diastereomer" refers to stereoisomers of two compounds that have the same planar structure, contain multiple asymmetric atoms with different configurations, and are not mirror images of each other.

As used herein, the term "solvate" refers to an association of one or more solvent molecules with the compound of the present application or the salt thereof. Solvents that form pharmaceutically acceptable solvates include, but are not limited to, water, isopropanol, ethanol, methanol, ethyl acetate, or acetic acid, etc.

In some embodiments, the mesenchymal-epithelial transition factor targeting peptide has the amino acid sequence as set forth in SEQ ID NO: 1.

As used herein, the term "amino acid" encompasses L-amino acids and D-amino acids.

In some embodiments, the derivative comprises any one or more modifications selected from the group consisting of amino acid modification, conservative amino acid substitution, or substitution of hydrogen in an amino acid residue, as compared with the mesenchymal-epithelial transition factor targeting peptide.

As used herein, the term "multiple" refers to two or more than two.

As used herein, the "derivative" of a polypeptide refers to a product obtained by performing amino acid modification, conservative amino acid substitution, and/or substitution of hydrogen in the amino acid residue on the basis of the mesenchymal-epithelial transition factor targeting peptide.

As used herein, the types of the term "amino acid modification" include, but are not limited to, N-terminal modification, C-terminal modification, and side-chain modification. The methods of "amino acid modification" include, but are not limited to, hydroxylation, carboxylation, alkylation, acylation, phosphorylation, sulfonation, amidation, aldehydation, alcoholation, mercaptoethylamination, esterification, glycosylation, etc.

As used herein, the term "conservative amino acid substitution" refers to the substitution of one amino acid with another amino acid having similar structure and/or chemical properties, such as substituting leucine with isoleucine or valine, substituting aspartic acid with glutamic acid, or substituting threonine with serine. Thus, "conservative amino acid substitution" means substituting the amino acid that is not important for the activity of the peptide, or substituting the amino acid with another amino acid having similar properties (e.g., acidic, basic, positively charged or negatively charged, polar or non-polar, etc.), such that even if the important amino acid is substituted, the activity of the peptide is not reduced. Conservative amino acid substitutions providing functionally similar amino acids are well known in the art. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W).

As used herein, when referring to the use of polypeptides, "amino acid" and "amino acid residue" have the same meaning. They refer to the remaining part of an amino acid after some of its groups are lost due to participating in the formation of connecting bonds when amino acids are linked via chemical bonds.

In some embodiments, the conservative amino acid substitutions include, for example, substitution of S with A or T in the amino acid sequence represented by RSPYANS, substitution of Y with F or W in the amino acid sequence represented by RSPYANS, and substitution of A with S or T in the amino acid sequence represented by RSPYANS.

In some embodiments, the substitution of hydrogen in an amino acid residue may be the substitution of hydrogen in the amino acid residue with any conventional substituent known in the art, including but not limited to alkyl, alkenyl, alkynyl, aryl, alkoxy, carboxyl, aldehyde, carbonyl, hydroxyl, halogen, cyano, acyl, sulfo, amino, mercapto, or nitro, etc.

In some embodiments, the taxane compound is one or more of a bicyclic taxane compound, a tricyclic taxane compound, a tetracyclic taxane compound, or a pentacyclic taxane compound.

As used herein, the term "taxane compound" mainly includes the compounds having a taxane backbone structure.

As used herein, the term "m cyclic taxane compound" refers to the taxane compound having a backbone with m fused rings; for example, the bicyclic taxane compound refers to the taxane compound having a backbone with two fused rings. The meaning of similar expressions can be deduced in the same way.

As used herein, the term "backbone" of the compound refers to the core structure of the compound, which can be substituted with multiple substituents on the backbone to form different compounds, specifically in the present application, different taxane compounds can be obtained.

As used herein, the term "fused" means that two rings share two or more atoms (including carbon atoms or heteroatoms).

As used herein, the term "ring" comprises saturated rings and unsaturated rings.

In some embodiments, the dicyclic taxane compound comprises a taxane compound with a 6-membered ring fused to a 12-membered ring. In some embodiments, the backbone of the taxane compound with a 6-membered ring fused to a 12-membered ring has the structure of Formula A. In some embodiments, the taxane compound with a 6-membered ring fused to a 12-membered ring can be substituted with various substituents based on the backbone of Formula A to form different types of taxane compounds with a 6-membered ring fused to a 12-membered ring.

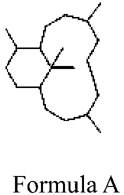

In some embodiments, the tricyclic taxane compound comprises one or more of taxane compounds with a 6-membered ring fused to an 8-membered ring fused to a 6-membered ring, a 6-membered ring fused to a 10-membered ring fused to a 6-membered ring, a 5-membered ring fused to a 7-membered ring fused to a 6-membered ring, or a 5-membered ring fused to a 6-membered ring fused to a 6-membered ring. In some embodiments, the backbone of the taxane compound with a 6-membered ring fused to an 8-membered ring fused to a 6-membered ring has the structure of Formula B. In some embodiments, the backbone of the taxane compound with a 6-membered ring fused to a 10-membered ring fused to a 6-membered ring has the structure of Formula C. In some embodiments, the backbone of the taxane compound with a 5-membered ring fused to a 7-membered ring fused to a 6-membered ring has the structure of Formula D. In some embodiments, the backbone of the taxane compound with a 5-membered ring fused to a 6-membered ring fused to a 6-membered ring has the structure of Formula E. In some embodiments, the taxane compound can be substituted with various substituents based on the backbones of Formulas B-E to form different types of tricyclic taxane compounds.

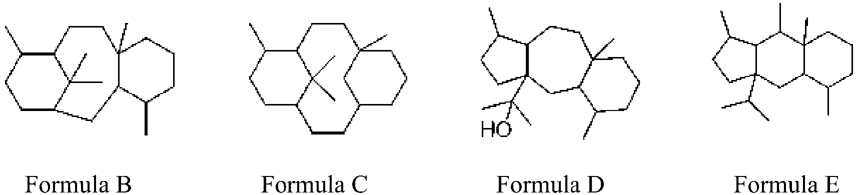

In some embodiments, the tetracyclic taxane compound comprises one or more of taxane compounds with a 6-membered ring fused to a 5-membered ring fused to a 5-membered ring fused to a 6-membered ring, or a 6-membered ring fused to an 8-membered ring fused to a 6-membered ring fused to a 6-membered ring. In some embodiments, the backbone of the taxane compound with a 6-membered ring fused to a 5-membered ring fused to a 5-membered ring fused to a 6-membered ring has the structure of Formula F. In some embodiments, the backbone of the taxane compound with a 6-membered ring fused to an 8-membered ring fused to a 6-membered ring fused to a 6-membered ring has the structure of Formula G. In some embodiments, the taxane compound can be substituted with various substituents based on the backbones of Formula F or G to form different types of tetracyclic taxane compounds.

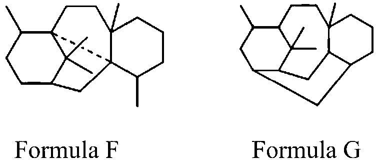

In some embodiments, the pentacyclic taxane compound comprises a taxane compound with a 6-membered ring fused to a 5-membered ring fused to a 5-membered ring fused to a 5-membered ring fused to a 6-membered ring. In some embodiments, the backbone of the taxane compound with a 6-membered ring fused to a 5-membered ring fused to a 5-membered ring fused to a 5-membered ring fused to a 6-membered ring has the structure of Formula H. In some embodiments, the taxane compound can be substituted with various substituents based on the backbone of Formula H to form different types of pentacyclic taxane compounds.

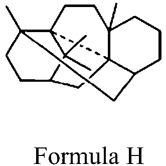

In some embodiments, the taxane compound comprises the structure represented by any one of the following 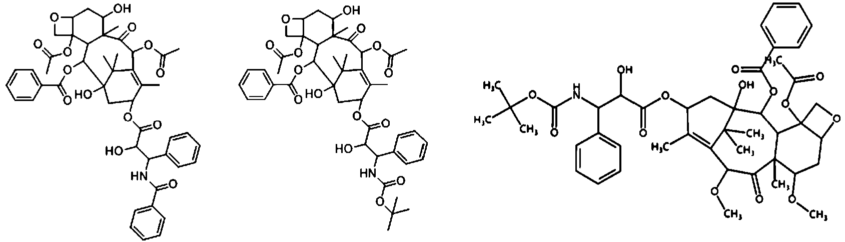 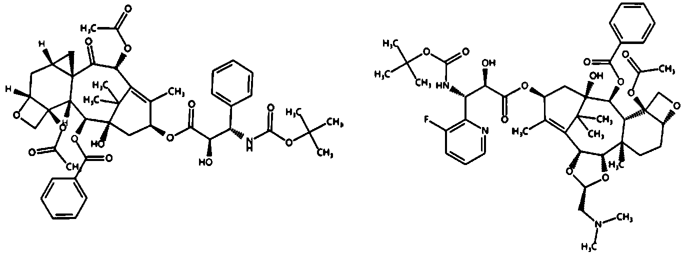 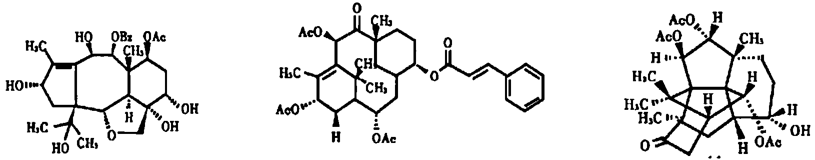 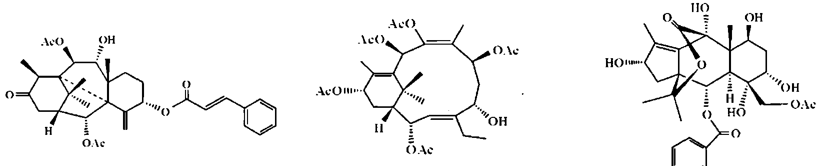 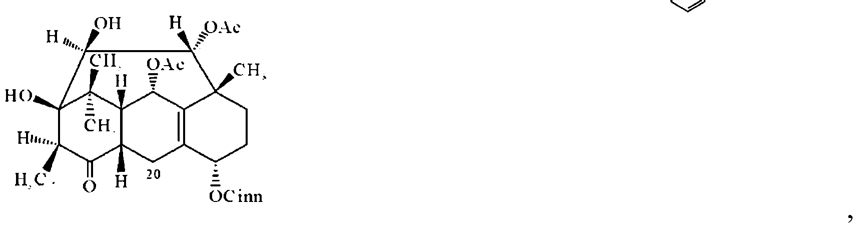 for more taxane compounds, refer to Fu Yan, et al. Research Progress of Taxane Compounds [J]. Natural Product Research and Development, 2004. Vol. 16, No. 3, P258-264; Huo Changhong, et al. The Proton Nuclear Magnetic Resonance Spectral Characteristics of Natural Taxane Compounds [J]. Chinese Journal of Magnetic Resonance, 2007, Vol. 24, No. 1, P101-118; Wang Chunlin, et al. Research Progress of Taxane Compounds [J]. Hebei Medical Journal, 2005. In some embodiments, any taxane compound known in the art can be incorporated into the present application and serve as the taxane compound described herein.

In some embodiments, the taxane compound comprises one or more of paclitaxel, docetaxel, cabazitaxel, larotaxel, or tesetaxel, and optionally, the taxane compound comprises one or more of paclitaxel or docetaxel. In some embodiments, the taxane compound is paclitaxel. In some embodiments, the taxane compound is docetaxel.

Paclitaxel, docetaxel, cabazitaxel, larotaxel, and tesetaxel all have the backbone structure of a 6-membered ring fused to an 8-membered ring fused to a 6-membered ring, and compounds with the same backbone have similar biological activities.

In some embodiments, the linker is CO(O)C(CH₂)ₙC(O)OH, n is an integer from 2 to 6. In some embodiments, the linker is CO(O)C(CH₂)ₙC(O)OH, n is selected from 2, 3, 4, 5, or 6.

In some embodiments, the linker is succinic acid, glutaric acid, adipic acid, pimelic acid, or suberic acid.

As used herein, the term "linker" refers to a structure that connects the polypeptide and the taxane compound.

As used herein, the term "succinic acid" refers to a compound with the chemical structure of HOC(O)CH₂CH₂C(O)OH.

In some embodiments, the linker is connected to the polypeptide by forming an amide bond. In specific embodiments, the carboxyl group of the linker forms an amide bond with the amino group of the polypeptide, the linker is connected to the polypeptide via the amide bond. In some embodiments, the carboxyl group of the linker forms an amide bond with the amino group of the N-terminal amino acid in the polypeptide, the amino group of the N-terminal amino acid is not a side-chain amino group. In some embodiments, the carboxyl group of the linker forms an amide bond with the non-side-chain amino group of arginine in the polypeptide.

In some embodiments, the linker is connected to the taxane compound by forming an ester bond. In specific embodiments, the carboxyl group of the linker forms an ester bond with the hydroxyl group of the taxane compound, the linker is connected to the taxane compound via the ester bond. In some embodiments, the carboxyl group of the linker forms an ester bond with the hydroxyl group at the 2'-position of the taxane compound.

In some embodiments, the polypeptide conjugate has the structure of the following Formula (II), (III), or (IV): 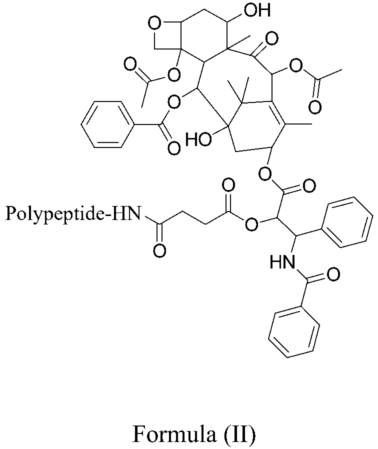 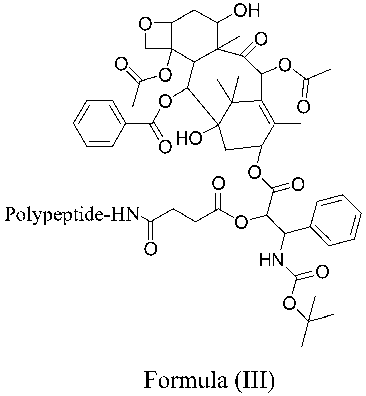 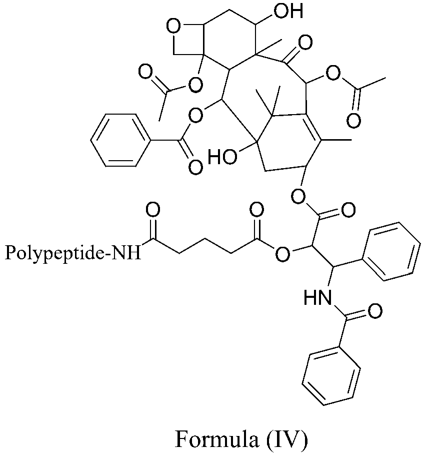 the polypeptide has the amino acid sequence as set forth in SEQ ID NO: 1 or a derivative thereof, the carboxyl group of the linker is connected to the amino group of the N-terminal amino acid of the polypeptide by forming an amide bond.

### Preparation Method of polypeptide conjugate

According to one embodiment of the present application, the present application provides a method for preparing the polypeptide conjugate as described in the present application, the method comprising:
(1) Providing the polypeptide;
(2) Linking the taxane compound and the linker to obtain the linker-taxane compound;
(3) Mixing the polypeptide with the linker-taxane compound to perform a liquid-phase reaction, thereby obtaining the polypeptide conjugate.

In some embodiments, in step (1), the polypeptide described in the present application can be synthesized by any method known in the art. For example, the polypeptide can be synthesized via a solid-phase reaction or a liquid-phase reaction.

In some embodiments, the solid-phase reaction is Fmoc solid-phase polypeptide reaction. In some embodiments, the Fmoc solid-phase polypeptide reaction is an prior art for polypeptide synthesis in the art, and those skilled in the art can perform the synthesis according to actual conditions.

In some embodiments, according to the amino acid sequence of the polypeptide, the carboxyl terminal amino acid, e.g., Fmoc-carboxyl terminal amino acid-OH, is coupled to Wang resin. After removing the Fmoc, the remaining amino acids are sequentially coupled according to the amino acid sequence of the polypeptide, and the Fmoc needs to be removed after each coupling of a new amino acid, yielding a polypeptide resin. In some embodiments, the remaining amino acids are added in the form of Fmoc-amino acid-OH.

In some embodiments, the side chains of the amino acids optionally are protected with protecting groups. Specifically, the protecting groups of the amino acid side chain include tert-butyl (t-Bu), triphenylmethyl (Trt) or Pbf. In some embodiments, serine (Ser), tyrosine (Tyr), and threonine (Thr) may be protected with t-Bu. In some embodiments, asparagine (Asn) and glutamine (Gln) may be protected with Trt. In some embodiments, arginine (Arg) may be protected with Pbf.

In some embodiments, a cleavage reagent is added to the polypeptide resin to obtain the peptide. In some embodiments, the cleavage reagent comprises one or two of trifluoroacetic acid (TFA) or triisopropylsilane (Tis).

In some embodiments, in step (2), the reaction temperature of the taxane compound and the linker is room temperature, the reaction time is ≥ 3 h.

As used herein, the term "room temperature" refers to room temperature or ambient temperature. For example, room temperature may be 25°C±5°C, 23°C±2°C, or 20°C±5°C.

In some embodiments, in step (3), the temperature at which the linker-taxane compound reacts with the polypeptide is room temperature, the reaction time is 3 h-8 h or 4 h-6 h.

In some embodiments, the obtained polypeptide conjugate can be verified by HPLC, thin-layer chromatography, MR, melting point, mass spectrometry. In some embodiments, the obtained polypeptide conjugate can be verified by mass spectrometry and SDS-polyacrylamide gel electrophoresis.

### Pharmaceutical Composition

In a second aspect, the present application provides a pharmaceutical composition comprising the polypeptide conjugate as described in the present application, and a pharmaceutically acceptable carrier.

As used herein, the term "pharmaceutically acceptable carrier" refers to a pharmaceutically acceptable raw material, component or carrier that imparts form or consistency to the pharmaceutical composition. When mixed, each carrier is compatible with the other components of the pharmaceutical composition, so as to avoid significantly reducing the efficacy of the polypeptide conjugate of the present application when administered to a subject and avoid interactions that would result in pharmaceutically unacceptable drug components.

In some embodiments, the pharmaceutically acceptable carrier includes, but is not limited to, a diluent, a filler, a binder, a disintegrant, a lubricant, a glidant, a granulating agent, a coating agent, a wetting agent, a solvent, a co-solvent, a suspending agent, an emulsifier, a sweetener, a flavoring agent, a taste-masking agent, a coloring agent, an anti-caking agent, a humectant, a chelating agent, a plasticizer, a tackifier, an antioxidant, a preservative, a stabilizer, a surfactant, or a buffering agent, etc. Those skilled in the art can selectively use the pharmaceutically acceptable carriers according to the dosage form of the pharmaceutical composition.

In some embodiments, the polypeptide conjugate of the present application is formulated with one or more of the pharmaceutically acceptable carriers into a dosage form suitable for administration to a subject via a desired route of administration. In some embodiments, the dosage form of the pharmaceutical composition includes, but is not limited to tablets, capsules, caplets, pills, lozenges, powders, syrups, fermenting agents, suspensions, solutions, emulsions, transdermal patches, suppositories, inhalants, creams, ointments, lotions, pastes, sprays, injections or gels, etc.

### Use

In a third aspect, the present application provides a use of the polypeptide conjugate as described in the present application or the pharmaceutical composition as described in the present application in the preparation of a drug for preventing or treating diseases.

As used herein, the term "disease" refers to any change in the state of the body or some organs, which interrupts or interferes with the execution of functions and/or causes symptoms (such as discomfort, dysfunction, adverse stress or even death) in the affected person or those in contact with them.

As used herein, the term "treatment" refers to alleviating or improving diseases or disorders (i.e., slowing or arresting the development of the diseases or at least one clinical symptom); or alleviating or improving at least one physical parameter or biomarker associated with the diseases or disorders.

In some embodiments, the diseases are tumors.

In some embodiments, the tumors comprise solid tumors and/or hematological tumors.

In some embodiments, the tumors comprise one or more of the group consisting of thyroid cancer, esophageal cancer, lung cancer, non-small cell lung cancer, gastric cancer, liver cancer, cholangiocarcinoma, renal cancer, pancreatic cancer, intestinal cancer, colorectal cancer, bladder cancer, prostate cancer, breast cancer, uterine cancer, endometrial cancer, ovarian cancer, cervical cancer, fallopian tube cancer, skin cancer, brain tumor, glioma, glioblastoma, melanoma, osteosarcoma, sarcoma, lymphoma, Hodgkin's lymphoma, non-Hodgkin's lymphoma, multiple myeloma, leukemia, acute lymphoblastic leukemia, chronic lymphocytic leukemia, acute myeloid leukemia, or chronic myeloid leukemia.

In some embodiments, the tumors comprise one or more of the group consisting of lung cancer, gastric cancer, liver cancer, ovarian cancer, prostate cancer, breast cancer, renal cancer, uterine cancer, thyroid cancer, pancreatic cancer, colorectal cancer, esophageal cancer, cholangiocarcinoma, skin cancer, lymphoma, multiple myeloma, glioma, melanoma, endometrial cancer, osteosarcoma, cervical cancer, bladder cancer, or leukemia.

In some embodiments, the tumors comprise one or more of the group consisting of non-small cell lung cancer, pulmonary giant cell carcinoma, gastric cancer, liver cancer, ovarian cancer, esophageal cancer, colon cancer, breast cancer, cholangiocarcinoma, osteosarcoma, cervical cancer, melanoma, bladder transitional cell carcinoma, pancreatic cancer, glioblastoma, chronic myeloid leukemia cells, or lymphoma.

### Treatment Method

According to one embodiment of the present application, the present application provides a method for preventing or treating diseases, and the method comprises administering the polypeptide conjugate as described in the present application or the pharmaceutical composition as described in the present application to a subject in need.

As used herein, the term "subject" refers to primates (e.g., humans), dogs, rabbits, guinea pigs, pigs, rats, and mice. In some embodiments, the subjects are the primates. In some specific embodiments, the subjects are humans.

As used herein, the subject is "in need of" such treatment if the subject benefits biologically, medically, or in terms of quality of life from the treatment.

As used herein, the term "therapeutically effective dose" refers to an amount that results in a benefit or treats the disease compared to a corresponding subject not receiving that amount, but the amount is sufficiently low within the scope of reasonable medical judgment to avoid serious side effects. The therapeutically effective dose of the polypeptide conjugate will vary with factors such as the specific polypeptide conjugate selected (e.g., considering the potency, efficacy, and half-life of the polypeptide conjugate); the route of administration selected; the disease being treated; the severity of the disease being treated; the age, body type, weight, and physical condition of the patient being treated; the medical history of the patient being treated; the duration of treatment; the nature of concurrent treatment; the desired therapeutic effect, etc., but can still be determined by those skilled in the art in a conventional manner.

In some embodiments, the diseases are tumors.

In some embodiments, the tumors comprise solid tumors and/or hematological tumors.

In some embodiments, the tumors comprise one or more of the group consisting of thyroid cancer, esophageal cancer, lung cancer, non-small cell lung cancer, gastric cancer, liver cancer, cholangiocarcinoma, renal cancer, pancreatic cancer, intestinal cancer, colorectal cancer, bladder cancer, prostate cancer, breast cancer, uterine cancer, endometrial cancer, ovarian cancer, cervical cancer, fallopian tube cancer, skin cancer, brain tumor, glioma, glioblastoma, melanoma, osteosarcoma, sarcoma, lymphoma, Hodgkin's lymphoma, non-Hodgkin's lymphoma, multiple myeloma, leukemia, acute lymphoblastic leukemia, chronic lymphocytic leukemia, acute myeloid leukemia, or chronic myeloid leukemia.

In some embodiments, the tumors comprise one or more of the group consisting of lung cancer, gastric cancer, liver cancer, ovarian cancer, prostate cancer, breast cancer, renal cancer, uterine cancer, thyroid cancer, pancreatic cancer, colorectal cancer, esophageal cancer, cholangiocarcinoma, skin cancer, lymphoma, multiple myeloma, glioma, melanoma, endometrial cancer, osteosarcoma, cervical cancer, bladder cancer, or leukemia.

In some embodiments, the tumors comprise one or more of the group consisting of non-small cell lung cancer, pulmonary giant cell carcinoma, gastric cancer, liver cancer, ovarian cancer, esophageal cancer, colon cancer, breast cancer, cholangiocarcinoma, osteosarcoma, cervical cancer, melanoma, bladder transitional cell carcinoma, pancreatic cancer, glioblastoma, chronic myeloid leukemia cells, or lymphoma.

The various embodiments described above with respect to the polypeptide conjugate, the polypeptide, the taxane compound, and the linker of the present application are also applicable to the pharmaceutical composition, the use, and the method of the present application (provided that they are not inherently contradictory to each other), and all various embodiments formed by such combination are deemed to be part of the present application.

The technical solutions of the present application will be described more clearly and explicitly below by way of illustration and in conjunction with embodiments. It should be understood that the embodiments are for illustrative purposes only and are not intended to limit the protection scope of the present application. The scope of protection of the present application is limited only by the claims. Therefore, those of ordinary skill in the art should recognize that various changes and modifications can be made to the embodiments described herein without departing from the scope of the present application. Similarly, for clarity and conciseness, descriptions of well-known functions and structures are omitted in the following description.

### Examples

Unless otherwise specified, the reagents and instruments used in the following embodiments are all conventional commercially available products. Unless otherwise specified, the experiments are conducted under conventional conditions or conditions recommended by the manufacturer.

### Example 1: Preparation of Peptide-Conjugated Paclitaxel (PTX-AI)

### 1. Preparation of P1-AI Peptide

10 g of Wang resin (CAS: 1365700-43-1) with a substitution degree of 0.91 mmol/g was weighed, added to a polypeptide solid-phase reactor (glass instrument, custom-made by the manufacturer), and washed twice with DMF. After swelling the Wang resin with DMF for 60 minutes, 12.71 g of Fmoc-Ser(tBu)-OH, 1.35 g of HOBt, 1.73 g of DIC, and 0.11 g of DMAP were taken, 60 mL of DMF was used as the solvent, the mixture was added to the solid-phase reactor, and reacted at room temperature for 3 hours. After the reaction, the mixture was washed three times with DMF and three times with DCM;

A capping solution (9.29 g of acetic anhydride, 3.53 g of DIEA, and 0.22 g of DMAP dissolved in 60 mL of DMF) was added for capping for 1.5 h. After capping, the mixture was washed three times with DMF, three times with DCM, and three times with methanol, then shrunk and dried by suction to obtain Fmoc-Ser(tBu)-Wang resin, the substitution value was detected to be 0.68 mmol/g;

2 g (1.36 mmol) of Fmoc-Ser(tBu)-Wang resin with a substitution value of 0.68 mmol/g was weighed, added to the solid-phase reactor, washed twice with DMF, the resin was swollen with DMF for 30 minutes, then dried by suction, and washed four times with DMF. 20% piperidine/DMF (100 mL/400 mL) was added to remove Fmoc twice, with the Fmoc removal times being 3 min and 7 min respectively, and the mixture was washed six times with DMF after the reaction was complete;

Fmoc-Asn(Trt)-OH (3 eq) and HOBt (3.6 eq) were weighed and dissolved in DMF, DIC (3.6 eq) was added under an ice bath at 0°C for activation for 4 min, the mixture was added to the solid-phase reactor after the activation was complete, and reacted at 25°C under nitrogen protection for 1 h. Amino detection was performed using the ninhydrin method (ninhydrin: phenol: piperidine = 2:1:1). If the resin was colorless and transparent, the reaction was complete; if the resin developed color, the reaction was incomplete, and the coupling reaction time needed to be extended (e.g., 0.5-1 h) until the resin was colorless and transparent as detected by the ninhydrin method. The mixture was dried by suction, washed four times with DMF, 20% piperidine/DMF (100 mL/400 mL) was added to remove Fmoc twice, with the Fmoc removal times being 3 min and 7 min respectively, and the mixture was washed six times with DMF after the reaction was complete;

The above steps were repeated, and each amino acid was sequentially added according to the peptide sequence. Fmoc-Ala-OH, Fmoc-Tyr(tBu)-OH, Fmoc-Pro-OH, Fmoc-Ser(tBu)-OH, and Fmoc-Arg(Pbf)-OH was coupled in sequence according to the amino acid sequence of SEQ ID NO:1. HOBt/DIC was used as a condensing agent for amino acid coupling, and each reaction lasted for 0.5 h. After each coupling, the resin was detected to be colorless and transparent by the ninhydrin method, and then the Fmoc protecting group was removed with 20% piperidine/DMF solution (100 mL/400 mL). The obtained product was washed four times with DMF, three times with DCM, and three times with MeOH, and dried to obtain P1-AI peptide resin.

The P1-AI peptide resin was placed in a cleavage reactor, and a cleavage reagent was added at a ratio of 10 mL per gram of resin. The ratio of the cleavage reagent was TFA: Tis: water = 95:2.5:2.5 (V/V/V). After stirring and reacting at room temperature for 2.0 h, the product was filtered with a funnel with fritted disc, the filtrate was collected, washed three times with a small amount of TFA, and the filtrates were combined, and concentrated under reduced pressure. The concentrated solution was added to cooled methyl tert-butyl ether for precipitation, the solid was precipitated, centrifuged, and washed with methyl tert-butyl ether and centrifuged four times, then vacuum-dried to obtain a crude product of the P1-AI linear peptide with a purity of 60.8% and a yield of 37.21%. Preparation purification was performed by HPLC, the chromatographic column was a reverse-phase C18 chromatographic column, the detection wavelength is 220 nm, 0.1% (v/v) TFA aqueous solution and acetonitrile were used as mobile phases A and B, for purification and elution, and the eluent of the target peak was collected, concentrated by rotary evaporation, and lyophilized to obtain the P1-AI polypeptide with a purity >99%.

### 2. Preparation of The Linker-Paclitaxel

1.20 g of paclitaxel (PTX, Anhui Zesheng Technology Co., Ltd.) was reacted with 0.21 g of succinic anhydride pyrimidine (Anhui Zesheng Technology Co., Ltd.) at room temperature for 3 hours to obtain succinyl-paclitaxel.

### 3. The Polypeptide Conjugate

0.88 g of succinyl-paclitaxel was reacted with 0.53 g of EDC (Anhui Zesheng Technology Co., Ltd.)/0.21 g of NHS (Shanghai Aladdin Biochemical Technology Co., Ltd.) dissolved in DCM at room temperature for 9 h to obtain succinyl-paclitaxel-NHS activated ester. 0.51 g of succinyl-paclitaxel-NHS activated ester and 0.58 g of the P1-AI polypeptide were dissolved in a solvent (water: N-methylpyrrolidone = 1:2.5, V:V), and triethylamine was added to adjust the pH to weakly alkaline, and the reaction was carried out at room temperature for 4 h to obtain 0.32 g of PTX-AI, which was then lyophilized. The purity of the PTX-AI was 96.7%, and the yield was 37.21%. During the reaction, the extent of the coupling reaction was monitored by HPLC.

### 4. Preparation of Other Polypeptide Conjugates

A polypeptide-conjugated docetaxel (DOC-AI) was obtained using the aforementioned method, with the only difference being that paclitaxel was replaced with docetaxel (Anhui Zesheng Technology Co., Ltd.).

A polypeptide PTX-5-AI was obtained using the aforementioned method, with the only difference being that succinic acid in PTX-AI was replaced with glutaric acid (Anhui Zesheng Technology Co., Ltd.).

### Example 2: Detection of Tumor Proliferation Inhibitory Effect of The Polypeptide Conjugates

Human highly metastatic hepatocellular carcinoma cells HCCLM3, human highly metastatic gastric cancer cells MKN45, human gastric cancer cells BGC-823 and MGC-803, non-small cell lung cancer cells A549, human pancreatic cancer cells ASPC-1, human colon cancer cells LoVo, human ovarian cancer cells A2780, human cervical cancer cells SiHa, human malignant melanoma cells SK-mel-2 and M14, human osteosarcoma cells Saos-2, human bladder transitional cell carcinoma cells T24, human brain glioblastoma cells U87-MG, human lymphoma cells Raji, and human epidermal cancer cells A431 were selected for the experiment. Among them, HCCLM3 cells, MKN45 cells, BGC-823 cells, MGC-803 cells, A431 cells and A549 cells were provided by Nanjing Anji Biotechnology Co., Ltd., and the other cells were provided by Nanjing Ouji Pharmaceutical Technology Service Co., Ltd.

The aforementioned tumor cells were preserved in a medium containing 10% fetal bovine serum and 1% penicillin-streptomycin, and incubated at 37°C with 5% CO₂ and 95% humidity. The aforementioned tumor cells were cultured in T75 flasks with an experimental cell density of 80%. The aforementioned tumor cell lines were mycoplasma-negative.

Tumor cells in the logarithmic growth phase were taken for the experiment. The cells were digested, counted, and prepared into a cell suspension with a concentration of 1×10⁵ cells/mL, which was then inoculated into a 96-well plate (100 µL per well). The 96-well plate was incubated in a 37°C, 5% CO₂ incubator for 24 hours. For different tumor cells, the administration groups were added with PTX-AI, DOC-AI, or PTX-5-AI at concentrations of 10 µM, 1 µM, 100 nM, 10 nM, 1 nM, 0.1 nM, and 0.01 nM per well, respectively; the positive control groups were added with PTX or DOC at concentrations of 10 µM, 1 µM, 100 nM, 10 nM, 1 nM, 0.1 nM, 0.01 nM per well, respectively; and the blank group was added with 100 µL of basal medium per well, and each group was set with 3 replicate wells. After incubating the plate in the incubator for 72 h, the cell morphology of each group was observed under a microscope. Then, 10 µL of CCK8 solution (Nanjing Enjing Biotechnology Co., Ltd.) was added to each well, and the plate was further incubated in the cell incubator for 4 h. After incubation, the absorbance was measured at 450 nm, and the tumor cell proliferation inhibition rate was calculated. The efficiency ratio was the ratio of the IC₅₀ value of the free drug to that of the polypeptide conjugate.

**Table 1 Tumor cell proliferation inhibition rates of polypeptide conjugates**

| Cell Lines | DOC IC₅₀ (nM) | DOC-AI IC₅₀ (nM) | Efficiency Ratios | PTX IC₅₀ (nM) | PTX-AI IC₅₀ (nM) | Efficiency Ratios |
|---|---|---|---|---|---|---|
| HCCLM3 (human highly metastatic hepatocellular carcinoma cells) | 430.6 | 264.2 | 1.63 | 9.40 | 2.54 | 3.70 |
| A549 (non-small cell lung cancer cells) | 33.5 | 24.1 | 1.39 | 541.40 | 264.20 | 2.05 |
| A2780 (human ovarian cancer cells) | - | - | - | 0.19 | 0.17 | 1.11 |
| LoVo (human colon cancer cells) | 992.5 | 837.6 | 1.18 | - | - | - |
| Saos-2 (human osteosarcoma cells) | - | - | - | 0.77 | 0.53 | 1.47 |
| SiHa (human cervical cancer cells) | 0.2349 | 0.0603 | 3.90 | - | - | - |
| SK-mel-2 (human malignant melanoma cells) | 0.2143 | 0.2135 | 1.00 | - | - | - |
| T24 (human bladder transitional cell carcinoma cells) | - | - | - | 38.62 | 21.72 | 1.78 |
| M14 (human malignant melanoma cells) | 3.682 | 3.041 | 1.21 | - | - | - |
| U87-MG (human brain glioblastoma cells) | 5.400 | 2.631 | 2.05 | - | - | - |
| ASPC-1 (human pancreatic cancer cells) | 4.350 | 1.881 | 2.31 | - | - | - |
| Raji (human lymphoma cells) | - | - | - | 43.87 | 40.12 | 1.09 |

| Cell Lines | PTX IC₅₀ (nM) | | PTX-5-AI IC₅₀ (nM) | | Efficiency Ratios | |
|---|---|---|---|---|---|---|
| MKN45 (human highly metastatic gastric cancer cells) | 3.21 | | 1.94 | | 1.65 | |
| BGC-823 (human gastric cancer cells) | 8.11 | | 4.26 | | 1.90 | |
| MGC-803 (human gastric cancer cells) | 23.60 | | 18.59 | | 1.27 | |
| A431 (human epidermal cancer cells) | 0.17 | | 0.08 | | 2.13 | |

Results showed that, as shown in Table 1, the polypeptide conjugates DOC-AI, PTX-AI, and PXT-5-AI of the present application had good tumor proliferation inhibitory effects on various tumor cells.

### Example 3: Detection of Inhibitory Effect of Polypeptide Conjugates on Tumor Horizontal Migration

A549 cells in the logarithmic growth phase were taken, digested with trypsin to prepare a cell suspension, and 1 mL of the cell suspension was inoculated into a 24-well plate with 3.5×10⁵ cells per well. The 24-well plate was incubated in a 37°C, 5% CO₂ incubator for 24 h, then a scratch was made vertically along the horizontal line at the bottom of the plate using a 200 µL pipette tip (one scratch per well). The original medium was discarded, and the wells were washed twice with 1 mL PBS. A blank group, a positive drug group, and an administration group were set up. The blank group was given 1 mL of F-12K medium containing 5% FBS; the positive drug group was given 1 mL of 100 nM DOC; and the administration group was given 1 mL each of 100 nM, 10 nM, and 1 nM DOC-AI, respectively. The cell migration status was photographed at 0 h and 48 h using a microscope (model: CKX31SF, manufacturer: OLYMPUS CORPORATION) at a magnification of 100×. Meanwhile, ImageJ 1.74V was used for quantitative calculation of the number of migrated tumor cells, and the results were expressed as mean ± SD (n = 3). Statistical analysis was performed for each time point using t-test (**** indicated that the P value of the difference between the blank group and other conditions was <0.001).

Results showed that, as shown in A and B of Figure. 1, compared with the blank group, DOC-AI effectively inhibited the horizontal migration of A549 cells in a concentration-dependent manner. Meanwhile, the effective concentration of DOC-AI was much lower than that of DOC, for example, the horizontal migration rate of 1 nM DOC-AI was lower than that of 100 nM DOC, which proved that the polypeptide conjugates of the present application could greatly enhance the efficacy of the compound.

### Example 4: Detection of Inhibitory Effect of Polypeptide Conjugates on Tumor Vertical Migration

A549 cells in the logarithmic growth phase were taken, digested with trypsin to prepare a cell suspension, and 100 µL of the cell suspension was inoculated into Transwell chambers with 3.5×10⁴ cells per well. A blank group, a positive drug group, and an administration group were set up. The blank group was given 100 µL of F-12K medium without FBS; the positive drug group was given 100 µL of 10 nM DOC; and the administration group was given 100 µL each of 1 µM, 100 nM, 10 nM, and 1 nM DOC-AI, respectively. 600 µL of F-12K medium containing 10% FBS was added to the 24-well plate holding the chambers. After incubation in a 37°C, 5% CO₂ incubator for 48 h, the cells were fixed with methanol for 30 min and stained with crystal violet for 10 min. After staining, the cell migration status was photographed under a microscope (model: CKX31SF, manufacturer: OLYMPUS CORPORATION) at a magnification of 100×. Meanwhile, Photoshop 20.0.1 was used for quantitative calculation of the number of migrated tumor cells, and the results were expressed as mean ± SD (n = 3). Statistical analysis was performed for each time point using t-test (**** indicated that the P value of the difference between the blank group and other conditions was <0.001).

Results showed that, as shown in A and B of Figure 2, compared with the blank group, DOC-AI effectively inhibited the vertical migration of A549 cells in a concentration-dependent manner, with the effective concentration of DOC-AI as low as 1 nM. Moreover, the effect of DOC-AI was superior to that of DOC; for example, the vertical migration rate of 10 nM DOC-AI was lower than that of 10 nM DOC, which proved that the polypeptide conjugates of the present application could greatly enhance the efficacy of the compound.

### Example 5: Detection of Tumor-Inhibitory Activity of The Polypeptide Conjugates In Vivo

Non-small cell lung cancer A549 cells and human gastric cancer MKN-45 cells were preserved in F12-K medium containing 10% fetal bovine serum and 1% penicillin + streptomycin, and incubated at 37°C with 5% CO₂ and 95% humidity. The aforementioned cells were cultured in T175 flasks with an experimental cell density of 80%. The aforementioned cell lines were mycoplasma-negative. Both A549 cells and MKN-45 cells were provided by Nanjing Anji Biotechnology Co., Ltd.

### 5.1 Detection of In Vivo Tumor-Inhibitory Activity of DOC-AI

### 5.1.1 Non-small cell lung cancer A549 cells

Non-small cell lung cancer A549 cells in the vigorous growth stage were taken and inoculated subcutaneously into the right axilla of BALB/c nude mice (Nanjing Zhonghua Biotechnology Co., Ltd.) under sterile conditions, with a cell inoculation amount of 1×10⁷. The diameter of transplanted tumors in nude mice was measured with a vernier caliper. When the tumors grew to approximately 50 mm³, 30 tumor-bearing nude mice with good growth status and relatively uniform tumor size were selected and divided into 5 groups, with 6 mice in each group. The specific grouping and administration methods were shown in Table 2 below. The administration frequency was continuous administration for 4 days followed by an interval of 3 days, and this cycle was repeated until day 21. The anti-tumor effect in the nude mice was dynamically observed by measuring the tumor diameter. The tumor diameter was measured every other day, and the body weight of the nude mice was weighed simultaneously during the measurement of the tumor diameter. After 21 days of administration, the nude mice were euthanized, the tumor inhibition rate (%) was calculated, and the tumor masses were peeled off and weighed.

**Table 2 Detection of In Vivo Tumor-Inhibitory Activity of DOC-AI**

| Group | Dosage Of Administration | Initial Tumor Volume | Final Tumor Volume | Tumor Weight (mg) | Tumor Inhibition Rate (%) |
|---|---|---|---|---|---|
| Model group | 10 ml/kg normal saline | 91.79±44.26 | 768.47±228.60 | 730.32±222.24 | - |
| DOC | 6.20 µmol/Kg | 85.82±27.45 | 310.83±131.01** | 283.00±179.83 | 59.55 |
| DOC-AI | 6.20 µmol/Kg | 87.14±27.23 | 73.75±23.01** | 81.00±40.81**^{##} | 90.40 |
| DOC-AI | 12.40 µmol/Kg | 88.73±43.55 | 31.31±22.73**^{##} | 51.83±31.36**^{##} | 95.33 |
| DOC-AI | 24.80 µmol/Kg | 86.33±37.21 | 20.30±20.80**^{##} | 20.53±20.80**^{##} | 97.33 |

| | | | | | |
|---|---|---|---|---|---|
| Note: *P<0.05, **P<0.01 vs model group; #P<0.05, ##P<0.01 vs DOC group | | | | | |

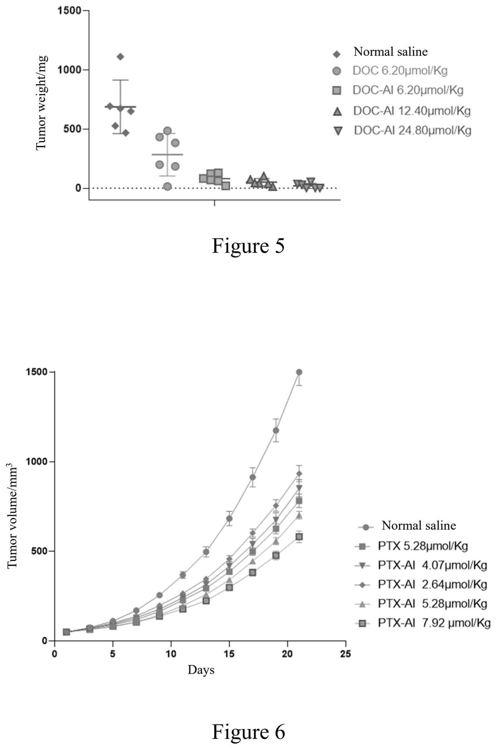

Results showed that, as could be seen from Table 2, Figure. 3 and Figure. 5, DOC-AI had a significant inhibitory effect on the growth of the non-small cell lung cancer A549 xenograft tumors in nude mice. At the same administration dose, for example, the tumor inhibition rate of 6.20 µM DOC was 59.55%, while that of DOC-AI at the same dose was as high as 90.40%, indicating that the tumor-inhibitory effect of the polypeptide conjugate DOC-AI was significantly superior to that of the positive drug DOC. Moreover, with the increase of dose, the inhibition rate of DOC-AI increased significantly, showing a dose-dependent manner. Meanwhile, as could be seen from Figure. 4, no significant weight loss was observed in nude mice of each group during the administration period.

### 5.1.2 Gastric cancer MKN45 cells

Gastric cancer MKN45 cells in the vigorous growth stage were taken and inoculated subcutaneously into the right axilla of BALB/c nude mice (Hangzhou Ziyuan Laboratory Animal Technology Co., Ltd.) under sterile conditions, with a cell inoculation amount of 1×10⁷. The diameter of transplanted tumors in nude mice was measured with a vernier caliper. When the tumors grew to approximately 50 mm³, 18 tumor-bearing nude mice with good growth status and relatively uniform tumor size were selected and divided into 3 groups, with 6 mice in each group. The specific grouping and administration methods were shown in Table 7 below. The administration frequency was once every 3 days, and this cycle was repeated until day 21. The anti-tumor effect in the nude mice was dynamically observed by measuring the tumor diameter. The tumor diameter was measured every other day, and the body weight of the nude mice was weighed simultaneously during the measurement of the tumor diameter. After 21 days of administration, the nude mice were euthanized, the tumor inhibition rate (%) was calculated, and the tumor masses were peeled off and weighed.

**Table 7 Results of Detection of In Vivo Tumor-Inhibitory Activity of DOC-AI**

| Group | Dosage of Administration | Initial Tumor Volume | Final Tumor Volume | Tumor Weight (g) | Tumor Inhibition Rate (%) |
|---|---|---|---|---|---|
| Model group | 10 ml/kg normal saline | 71.09±22.46 | 1012.53±301.08 | 1165.82±301.08 | - |
| DOC | 6.20 µmol/Kg | 73.29±35.78 | 256.67±391.25** | 578.82±391.25** | 73.76±13.15 |
| DOC-AI | 6.20 µmol/Kg | 70.50±21.27 | 191.31±54.48** | 204.34±54.48** | 84.25±9.15 |

| | | | | | |
|---|---|---|---|---|---|
| Note: *P<0.05, **P<0.01 vs model group; #P<0.05, ##P<0.01 vs DOC group | | | | | |

Results showed that, as could be seen from Table 7, and A and B of Figure. 9, DOC-AI had a significant inhibitory effect on the growth of gastric cancer MKN45 xenograft tumors in nude mice. At the same administration dose, for example, the tumor inhibition rate of 6.20 µmol/kg DOC was 73.76±13.15%, while that of DOC-AI at the same dose was as high as 84.25±9.15%, indicating that the tumor-inhibitory effect of the polypeptide conjugate DOC-AI was significantly superior to that of the positive drug DOC. Meanwhile, as could be seen from C of Figure. 9, no significant weight loss was observed in nude mice in the DOC-AI administration group during the administration period, whereas the nude mice in the positive drug DOC group showed weight loss, which indicated that DOC-AI had good safety.

### 5.2 Detection of Tumor-Inhibitory Activity of PTX-AI In Vivo

Human gastric cancer cells MKN-45 in the vigorous growth stage were taken and inoculated subcutaneously into the right axilla of BALB/c nude mice under sterile conditions, with a cell inoculation amount of 5×10⁶. The diameter of transplanted tumors in nude mice was measured with a vernier caliper. When the tumors grew to approximately 50mm³, 68 tumor-bearing nude mice with good growth status and relatively uniform tumor size were selected, and divided into 6 groups, including 10 mice in the model group and 8 mice in each of the remaining groups. The specific grouping and administration methods were shown in Table 3 below. The anti-tumor effect of the test substance was dynamically observed by measuring the tumor diameter. The tumor diameter was measured every other day, and the body weight of the nude mice was weighed simultaneously during the measurement of the tumor diameter. After 21 days of administration, the nude mice were euthanized, the tumor inhibition rate (%) was calculated, and the tumor masses were peeled off and weighed.

**Table 3 Detection of In Vivo Tumor-Inhibitory Activity of PTX-AI**

| Group | Dosage of Administration | Administration Frequency | Initial Tumor Volume | Final Tumor Volume | Tumor Weight (g) | **Tumor** Inhibition Rate (%) |
|---|---|---|---|---|---|---|
| Model group | 10 ml/kg normal saline | Once every other day | 49.2±6.0 | 1499.4±235.0 | 1.509±0.292 | - |
| PTX | 5.28 µmol/Kg | Once every 3 days | 51.4±3.5 | 782.0±105.9** | 0.791±0.151** | 47.6 |
| PTX-AI | 5.28 µmol/Kg | Continuous administration for 4 days a week | 50.3±3.1 | 700.6±59.6** | 0.705±0.066** | 53.3 |
| PTX-AI | 4.07 µmol/Kg | Once every other day | 49.1±2.4 | 852.1±137.3** | 0.861±0.126** | 42.9 |
| PTX-AI | 2.64 µmol/Kg | Once every other day | 49.6±5.5 | 934.0±125.0** | 0.961±0.119** | 36.3 |
| PTX-AI | 7.92 µmol/Kg | Once every other day | 50.9±5.9 | 579.8±91.9** | 0.591±0.099** | 60.8 |

| | | | | | | |
|---|---|---|---|---|---|---|
| Note: *P<0.05, **P<0.01 vs model group | | | | | | |

Results showed that, as could be seen from Table 3 and Figure. 6, the polypeptide conjugate PTX-AI had a significant inhibitory effect on the growth of human gastric cancer cell MKN45 xenograft tumors in nude mice. With the increase of dose, the inhibition rate of PTX-AI increased significantly, showing a dose-dependent manner. When the administration dose was 7.92 µmol/kg, the highest tumor inhibition rate of PTX-AI was 60.8%. Meanwhile, it could also be seen that at the same dose, the tumor-inhibitory activity of the polypeptide conjugate PTX-AI was superior to that of free PTX, where the difference in administration frequency between the free PTX and the polypeptide conjugate was due to the fact that the free PTX had relatively strong toxic and side effects on the body, and the administration frequency of the polypeptide conjugate would cause irreversible damage to the rats, affecting the measurement of subsequent data. This phenomenon also proved that the polypeptide conjugate of the present application had better safety due to its targeting property.

### Example 6: Detection of metabolism of polypeptide conjugates in liver microsomes

1.85 mg of DOC-AI (or 1.81 mg of PTX-AI) was precisely weighed, 1 mL of DMSO was added, dissolved and mixed well to obtain 1 mM stock solution. 1 mM stock solution was diluted with methanol to 100 µM working solution for sample incubation. 12.5 µL of 20 mg/mL liver microsomes (mouse-derived, Shanghai Quanyang Trading Co., Ltd., batch number: MIC255038; rat-derived, Shanghai Quanyang Trading Co., Ltd., batch number: MIC254034; dog-derived, Shanghai Quanyang Trading Co., Ltd., batch number: MIC257015; monkey-derived, Ruide Liver Disease Research (Shanghai) Co., Ltd., batch number: SYXS; human-derived, Shanghai Quanyang Trading Co., Ltd., batch number: 1210097), 432.5 µL of 100 mM PBS, 25 µL of 20 mM NADPH, and 25 µL of 100 mM MgCl₂ were added into a 96-well plate, and after mixing, pre-incubated at 37°C for 5 min. 5 µL of 100 µM working solution was added to the 96-well plate to initiate the reaction. At the set time points, 50 µL of the reaction sample was taken and placed in an EP tube containing 100 µL of ice-cold acetonitrile, vortexed for inactivation, and stored at -90°C for testing. The concentrations of the polypeptide conjugate DOC-AI and the dissociated DOC were quantitatively detected by the LC-MS/MS method.

**Table 4 Results of detection of DOC-AI metabolism in liver microsomes**

| Compound | Liver Microsome Concentration (mg/mL) | Related Parameters | | | | | | |
|---|---|---|---|---|---|---|---|---|
| | | R² | k | t_{½} (min) | CL_{int(mic)} (µL/min/mg) | CLᵢₙₜ₍ₗᵢᵥₑᵣ₎ (mL/min/kg) | Hepatic Extraction Ratio (E_{H}) | Clearance Rate Level |
| Mouse | | 0.996 | 0.0410 | 16.9 | 81.9 | 324 | 0.783 | High |
| Rat | | 0.995 | 0.0301 | 23.1 | 60.1 | 108 | 0.662 | Medium |
| Dog | 0.5 | 0.996 | 0.0230 | 30.2 | 45.9 | 66.2 | 0.682 | Medium |
| Monkey | | 0.995 | 0.0520 | 13.3 | 104 | 140 | 0.763 | High |
| Human | | 0.982 | 0.0130 | 53.3 | 26.0 | 23.4 | 0.531 | Medium |

**Table 5 Results of detection of PTX-AI metabolism in liver microsomes**

| Compound | Liver Microsome Concentration (mg/mL) | Related Parameters | | | | | | |
|---|---|---|---|---|---|---|---|---|
| | | R² | k | t_{½} (min) | CL_{int(mic)} (µL/min/mg) | CLᵢₙₜ₍ₗᵢᵥₑᵣ₎ (mL/min/kg) | Hepatic Extraction Ratio (E_{H}) | Clearance Rate Level |
| Mouse | | 0.986 | 0.0511 | 13.6 | 102 | 405 | 0.818 | High |
| Rat | | 0.998 | 0.0339 | 20.4 | 67.8 | 122 | 0.689 | Medium |
| Dog | 0.5 | 0.993 | 0.0283 | 24.5 | 56.6 | 81.4 | 0.725 | High |
| Monkey | | 0.997 | 0.0548 | 12.7 | 110 | 148 | 0.772 | High |
| Human | | 0.976 | 0.0148 | 46.9 | 29.5 | 26.6 | 0.562 | Medium |

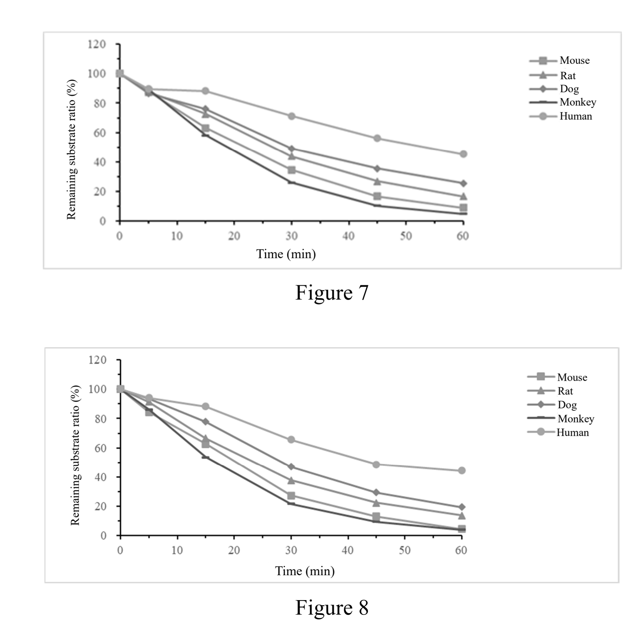

The in vitro metabolic incubation system of the liver microsomes was used to study the metabolic stability of polypeptide conjugates DOC-AI and PTX-AI in mice, rats, dogs, monkeys, and humans, and to analyze their metabolic half-lives and clearance rates in liver microsomal enzymes of different species, so as to provide a basis for the selection of animals in subsequent preclinical in vivo experiments. The results in Tables 4-5 and Figures. 7-8 showed that DOC-AI was a high-clearance drug in mice and monkeys, and a medium-clearance drug in rats, dogs, and humans. PTX-AI was a high-clearance drug in mice, dogs, and monkeys, and a medium-clearance drug in rats and humans. Medium-clearance drugs had good stability in organisms, would not be rapidly degraded by the body, and would not excessively accumulate in the body, thus avoiding excessive damage to the body.

### Example 7: Determination of solubility of polypeptide conjugates

Preparation of a series of standard solutions of DOC, DOC-AI, PTX, and PTX-AI:
2 mg each of DOC, DOC-AI, PTX, and PTX-AI were weighed and added into 2 mL volumetric flasks, methanol was added to the tick mark, and shaken well to obtain 1 mg/mL stock solution. 25, 50, 100, 200, 300, and 400 µL of the above stock solutions were precisely measured, added into 1 mL volumetric flasks, and diluted to the mark with methanol to obtain a series of standard solutions with concentrations of 25, 50, 100, 200, 300, and 400 µg/mL, respectively. The peak areas of the series of standard solutions were detected by HPLC (mobile phase: 0.065% TFA in water + 0.05% TFA; chromatographic column: YMC-Triart C18; wavelength: 220 nm) to construct a concentration-peak area standard curve, where the abscissa represents concentration and the ordinate represents peak area.

Preparation of supersaturated aqueous solutions of DOC, DOC-AI, PTX, and PTX-AI:
10 mg each of DOC, DOC-AI, PTX, and PTX-AI were weighed and added into 5 mL of ultrapure water, 1 mL of ultrapure water was added, sonicated for 15 min, and filtered through an organic filter membrane into a liquid phase vial. The peak area of the sample was detected by HPLC, and its saturation in water was calculated through the concentration-peak area standard curve. The results were shown in Table 6.

**Table 6 Results of detection of solubility of polypeptide conjugates in water**

| Sample | Peak Area | Solubility (mg/mL) | Fold Increase |
|---|---|---|---|
| DOC | 0.29 | 2.74×10⁻⁵ | - |
| DOC-AI | 35583.20 | 4.43 | 1.62×10⁵ |
| PTX | 2.64 | 1.2×10⁻⁴ | - |
| PTX-AI | 42.59 | 5.56×10⁻³ | 46.33 |

As could be seen from Figure. 10, the R² values of the concentration-peak area standard curves for DOC, DOC-AI, PTX, and PTX-AI were all greater than 0.99, indicating good fitting degree. As shown in Table 6, the solubility of DOC and PTX in water was 2.74×10⁻⁵ mg/mL and 1.2×10⁻⁴ mg/mL, respectively, which were almost insoluble in water. DOC-AI had good solubility in water, which was 4.43 mg/mL, and was 1.62×10⁵ times higher than that of the positive drug DOC; the solubility of PTX-AI was 46.33 times higher than that of the positive drug PTX.

Machulkin et al. coupled a PSMA-targeted ligand with DOC via a dipeptide linker to obtain PSMA-DOC, which had a solubility in water of 130 µg/mL (Machulkin AE, Uspenskaya AA, Zyk NY, et al. PSMA-targeted small-molecule docetaxel conjugate: Synthesis and preclinical evaluation. Eur J Med Chem. 2022;227:113936.), and this still had significant restrictions on the application of PSMA-DOC. However, the polypeptide conjugate of the present application had a solubility in water of 4.43 mg/mL, which was 1.62×10⁵ times higher than the solubility of DOC alone, and could greatly improve the application of DOC. The compound SNG-1005, formed by coupling PTX molecules with the 19-amino-acid peptide Angiopep-2, had completed Phase III clinical trials. However, SNG1005 still had insolubility when in contact with water, making its batch production difficult and forcing the postponement of related clinical trials. It could be seen from this that the polypeptide conjugate of the present application greatly increased the water solubility of DOC and PTX.

Although specific embodiments have been described, alternatives, modifications, variations, improvements, and substantial equivalents to the aforementioned embodiments may exist or be currently unforeseeable to the applicant or other persons skilled in the art. Therefore, the appended claims as filed and any claims that may be amended are intended to cover all such alternatives, modifications, variations, improvements, and substantial equivalents.

## Claims

1. A polypeptide conjugate having the structure of Formula (I):
polypeptide-linker-compound Formula (I)
wherein,
the polypeptide is a mesenchymal-epithelial transition factor targeting peptide or a derivative thereof,
the compound is a taxane compound or an enantiomer, a diastereoisomer, a solvate or a pharmaceutically acceptable salt thereof.

2. The polypeptide conjugate according to claim 1, wherein, the mesenchymal-epithelial transition factor targeting peptide has the amino acid sequence as set forth in SEQ ID NO: 1.

3. The polypeptide conjugate according to claim 1 or 2, wherein, the derivative comprises any one or more modifications selected from the group consisting of amino acid modification, conservative amino acid substitution, or substitution of hydrogen in an amino acid residue, as compared with the mesenchymal-epithelial transition factor targeting peptide.

4. The polypeptide conjugate according to any one of claims 1-3, wherein, the taxane compound is one or more of a bicyclic taxane compound, a tricyclic taxane compound, a tetracyclic taxane compound or a pentacyclic taxane compound, optionally, the bicyclic taxane compound comprises a taxane compound with a 6-membered ring fused to a 12-membered ring, the tricyclic taxane compound comprises one or more of taxane compounds with a 6-membered ring fused to an 8-membered ring fused to a 6-membered ring, a 6-membered ring fused to a 10-membered ring fused to a 6-membered ring, a 5-membered ring fused to a 7-membered ring fused to a 6-membered ring, or a 5-membered ring fused to a 6-membered ring fused to a 6-membered ring, the tetracyclic taxane compound comprises one or more of taxane compounds with a 6-membered ring fused to a 5-membered ring fused to a 5-membered ring fused to a 6-membered ring, or a 6-membered ring fused to an 8-membered ring fused to a 6-membered ring fused to a 6-membered ring, the pentacyclic taxane compound comprises a taxane compound with a 6-membered ring fused to a 5-membered ring fused to a 5-membered ring fused to a 5-membered ring fused to a 6-membered ring.

5. The polypeptide conjugate according to any one of claims 1-4, wherein, the taxane compound comprises one or more of paclitaxel, docetaxel, cabazitaxel, larotaxel, or tesetaxel, optionally, the taxane compound comprises one or more of paclitaxel or docetaxel.

6. The polypeptide conjugate according to any one of claims 1-5, wherein, the linker is CO(O)C(CH₂)ₙC(O)OH, n is an integer of 2-6, optionally, the linker is succinic acid, glutaric acid, adipic acid, pimelic acid, or suberic acid.

7. The polypeptide conjugate according to any one of claims 1-6, wherein, the linker is connected to the polypeptide by forming an amide bond, the linker is connected to the taxane compound by forming an ester bond.

8. The polypeptide conjugate according to any one of claims 1-7, wherein the polypeptide conjugate has the structure of the following Formula (II), (III), or (IV): 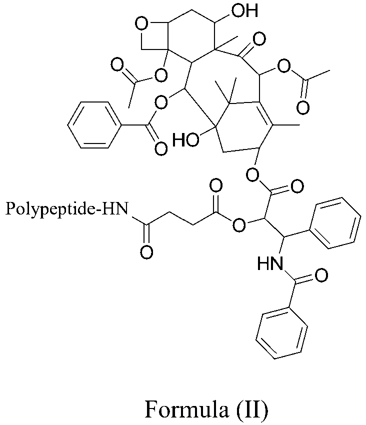 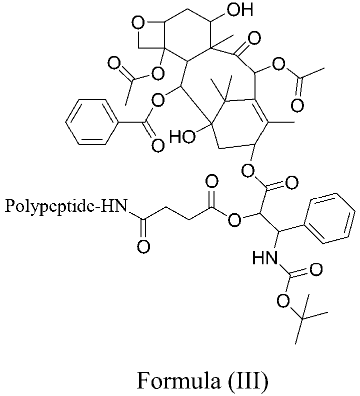 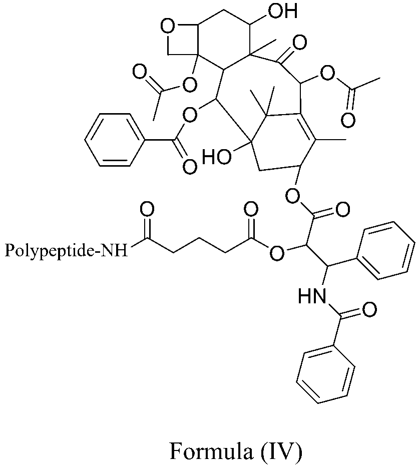 the polypeptide has the amino acid sequence as set forth in SEQ ID NO: 1 or a derivative thereof, the carboxyl group of the linker is connected to the amino group of the N-terminal amino acid of the polypeptide by forming an amide bond.

9. A pharmaceutical composition comprising the polypeptide conjugate according to any one of claims 1-8, and a pharmaceutically acceptable carrier.

10. A use of the polypeptide conjugate according to any one of claims 1-8 or the pharmaceutical composition according to claim 9 in the preparation of a drug for preventing or treating diseases, optionally, the diseases comprise tumors, more optionally, the tumors comprise one or more of the group consisting of thyroid cancer, esophageal cancer, lung cancer, non-small cell lung cancer, gastric cancer, liver cancer, cholangiocarcinoma, renal cancer, pancreatic cancer, intestinal cancer, colorectal cancer, bladder cancer, prostate cancer, breast cancer, uterine cancer, endometrial cancer, ovarian cancer, cervical cancer, fallopian tube cancer, skin cancer, brain tumor, glioma, glioblastoma, melanoma, osteosarcoma, sarcoma, lymphoma, Hodgkin's lymphoma, non-Hodgkin's lymphoma, multiple myeloma, leukemia, acute lymphoblastic leukemia, chronic lymphocytic leukemia, acute myeloid leukemia, or chronic myeloid leukemia.
